(19) Europäisches Patentamt
European Patent Office
Office européen des brevets

(11) Veröffentlichungsnummer: **0 198 271 B1**

(12) **EUROPÄISCHE PATENTSCHRIFT**

(45) Veröffentlichungstag der Patentschrift: **09.06.93**

(51) Int. Cl.$^5$: **C07K 5/02**, C07K 5/06, C07D 233/64, A61K 37/64, A61K 31/415, A61K 31/16

(21) Anmeldenummer: **86103944.4**

(22) Anmeldetag: **22.03.86**

Die Akte enthält technische Angaben, die nach dem Eingang der Anmeldung eingereicht wurden und die nicht in dieser Patentschrift enthalten sind.

# (54) Peptide.

(30) Priorität: **03.04.85 DE 3512128**

(43) Veröffentlichungstag der Anmeldung:
**22.10.86 Patentblatt 86/43**

(45) Bekanntmachung des Hinweises auf die Patenterteilung:
**09.06.93 Patentblatt 93/23**

(84) Benannte Vertragsstaaten:
**AT BE CH DE FR GB IT LI NL SE**

(56) Entgegenhaltungen:
**EP-A- 0 081 783** **EP-A- 0 104 964**
**EP-A- 0 111 266** **EP-A- 0 155 809**
**EP-A- 0 165 151** **WO-A-84/03044**

(73) Patentinhaber: **MERCK PATENT GESELLSCHAFT MIT BESCHRÄNKTER HAFTUNG**
**Frankfurter Strasse 250 Postfach 4119**
**W-6100 Darmstadt(DE)**

(72) Erfinder: **Raddatz, Peter, Dr.**
**Grafenstrasse 37**
**W-6100 Darmstadt(DE)**
Erfinder: **Hölzemann, Günter, Dr.**
**Weedring 5**
**W-6104 Seeheim 1(DE)**
Erfinder: **Jonczyk, Alfred, Dr.**
**Jungfernstrasse 30**
**W-6100 Darmstadt 12(DE)**
Erfinder: **Schmitges, Claus, Dr.**
**Karolingerstrasse 5**
**W-6114 Gross-Umstadt(DE)**
Erfinder: **Minck, Klaus-Otto, Dr.**
**Büchestrasse 8**
**W-6105 Ober-Ramstadt(DE)**
Erfinder: **Radunz, Hans-Eckart, Dr.**
**Am Klingenteich 5**
**W-6109 Mühltal(DE)**
Erfinder: **Sombroek, Johannes, Dr.**
**Robert-Koch-Strasse 32**
**W-6100 Darmstadt-Eberstadt(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die Erfindung betrifft neue Peptide der Formel I

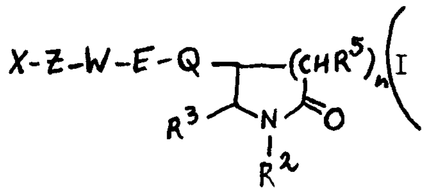

I

worin

| | |
|---|---|
| X | H, $R^{1-}$0-$C_mH_{2m}$-C0-, $R^{1-}C_mH_{2m}$-0-C0-, $R^1$-$C_mH_{2m}$-C0-, $R^1$-$S0_2$-, ($R^1$-$C_mH_{2m}$)-L($R^{1-}C_pH_{2p}$)-$C_rH_{2r}$-C0-, H-($NHCH_2CH_2$)$_m$-NH-$CH_2$CO- oder 9-Fluorenyl-$C_mH_{2m}$-O-CO, |
| Z | 0 bis 4 peptidartig miteinander verbundene Aminosäurereste ausgewählt aus der Gruppe bestehend aus |

Abu, Ada, Ala, Arg, Asn, Bia, Dab, Gln, Gly, His, N(im)-Alkyl-His, Ile, Leu, tert.-Leu, Lys, Met, Nbg, Nle, Orn, Phe, Pro, Ser, Thr, Tic, Trp, Tyr und Val,

| | |
|---|---|
| W | -$NR^2$-$CHR^3$-$CHR^4$-($CHR^5$)$_n$-C0- |
| E | 0 bis 2 peptidartig miteinander verbundene Aminosäurereste ausgewählt aus der Gruppe bestehend aus Abu, Ala, Ile, Leu, Met, Nle und Val, |
| $R^1$ und $R^3$ | jeweils A, Ar, Ar-alkyl, unststituiertes oder ein- oder mehrfach durch Alkyl, Alkoxy und/oder Hal substituiertes Cycloalkyl mit 3-7 C-Atomen, Cycloalkyl- alkyl mit 4-11 C-Atomen, Bicycloalkyl oder Tricycloalkyl mit jeweils 7-14 C-Atomen, Bicycloalkyl-alkyl oder Tricycloalkyl-alkyl mit jeweils 8-18 C-Atomen, |
| $R^2$ und $R^5$ | jeweils H oder A, |
| $R^4$ | OH oder $NH_2$, |
| L | CH oder N, |
| m, p und r | jeweils 0, 1, 2, 3, 4, oder 5, |
| n | 1 oder 2, |
| Q | 0 oder NH, |
| Ar | unsubstituiertes oder ein- oder mehrfach durch A, A0, Hal, $CF_3$, 0H und/oder $NH_2$ substituiertes Phenyl oder unsubstituiertes Naphthyl, |
| Hal | F, Cl, Br oder J und |
| A | Alkyl mit 1-8 C-Atomen bedeuten, |

worin ferner an Stelle einer oder mehrerer -NH-C0-Gruppen auch eine oder mehrere -N(Alkyl)-C0-Gruppen stehen können,
wobei die Reste $R^2$, $R^3$ und $R^5$ sowie die Parameter n gleich oder verschieden sein können,
sowie deren Salze.

Ähnliche Verbindungen sind aus der EP-A-77028 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze sehr wertvolle Eigenschaften besitzen. Vor allem hemmen sie die Aktivität des menschlichen Plasmarenins. Diese Wirkung kann z. B. nach der Methode von F. Fyhrquist et. al., Clin.Chem. 22, 250-256 (1976), nachgewiesen werden. Bemerkenswert ist, daß diese Verbindungen sehr spezifische Hemmer des Renins sind; für die Hemmung anderer Aspartylproteinasen (z. B. Pepsin und Kathepsin D) sind wesentlich höhere Konzentrationen dieser Verbindungen notwendig.

Die Verbindungen können als Arzneimittelwirkstoffe in der Human- und Veterinärmedizin eingesetzt werden, insbesondere zur Prophylaxe und zur Behandlung von Herz-, Kreislauf- und Gefäßkrankheiten, vor allem von Hypertonie, Herzinsuffizienz und Hyperaldosteronismus. Außerdem können die Verbindungen zu

diagnostischen Zwecken verwendet werden, um bei Patienten mit Hypertonie oder Hyperaldosteronismus den möglichen Beitrag der Reninaktivität zur Aufrechterhaltung des pathologischen Zustands zu bestimmen.

Die vor- und nachstehend aufgeführten Abkürzungen von Aminosäureresten stehen für die Reste -NH-CHR-CO- (worin R die für jede Aminosäure bekannte spezifische Bedeutung hat) folgender Aminosäuren:

| | |
|---|---|
| Abu | 2-Aminobuttersäure |
| Ada | Adamantylalanin |
| Ala | Alanin |
| Arg | Arginin |
| Asn | Asparagin |
| Bia | Benzimidazolylalanin |
| Dab | 2,4-Diaminobuttersäure |
| Gln | Glutamin |
| Gly | Glycin |
| His | Histidin |
| N(im)-Alkyl-His | in 1- oder 3-Stellung des Imidazolrings durch A substituiertes Histidin |
| Ile | Isoleucin |
| Leu | Leucin |
| tert.-Leu | tert.-Leucin |
| Lys | Lysin |
| Met | Methionin |
| Nbg | (2-Norbornyl)-glycin |
| Nle | Norleucin |
| N-Me-His | N-Methyl-histidin |
| N-Me-Phe | N-Methyl-phenylalanin |
| Orn | Ornithin |
| Phe | Phenylalanin |
| Pro | Prolin |
| Ser | Serin |
| Thr | Threonin |
| Tic | Tetrahydroisochinolin-3-carbonsäure |
| Trp | Tryptophan |
| Tyr | Tyrosin |
| Val | Valin |

Ferner bedeutet nachstehend:

| | |
|---|---|
| BOC | tert.-Butoxycarbonyl |
| imi-BOM | Benzyloxymethyl in 1-Stellung des Imidazolrings |
| CBZ | Benzyloxycarbonyl |
| DNP | 2,4-Dinitrophenyl |
| FMOC | 9-Fluorenylmethoxycarbonyl |
| imi-DNP | 2,4-Dinitrophenyl in 1-Stellung des Imidazolrings |
| OMe | Methylester |
| POA | Phenoxyacetyl |
| DCCI | Dicyclohexylcarbodiimid |
| HOBt | 1-Hydroxybenzotriazol. |

Sofern die vorstehend genannten Aminosäuren in mehreren enantiomeren Formen auftreten können, so sind vor- und nachstehend, z. B. als Bestandteil der Verbindungen der Formel I, alle diese Formen und auch ihre Gemische (z. B. die DL-Formen) eingeschlossen. Die L-Formen sind bevorzugt. Sofern nachstehend einzelne Verbindungen aufgeführt sind, so beziehen sich die Abkürzungen dieser Aminosäuren jeweils auf die L-Form, sofern nicht ausdrücklich etwas anderes angegeben ist.

Vor- und nachstehend haben die Reste bzw. Parameter X, Z, W, E, $R^1$ bis $R^6$, L, m, n, p, r, t, Q, Ar, Hal und A die bei Formel I angegebenen Bedeutungen, falls nicht ausdrücklich etwas anderes angegeben ist.

In den vorstehenden Formeln hat A 1 - 8, vorzugsweise 1,2,3 oder 4 C-Atome. A bedeutet vorzugsweise Methyl, Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl.

Cycloalkyl bedeutet vorzugsweise Cyclopropyl, Cyclobutyl, Cyclopentyl, Cyclohexyl oder Cycloheptyl, aber auch z. B. 1-, 2- oder 3-Methylcyclopentyl, 1-, 2-, 3- oder 4-Methylcyclohexyl.

Dementsprechend bedeutet Cycloalkyl-alkyl vorzugsweise Cyclopropylmethyl, 2-Cyclopropylethyl, Cyclobutylmethyl, 2-Cyclobutylethyl, Cyclopentylmethyl, 2-Cyclopentylethyl, Cyclohexylmethyl, 2-Cyclohexylethyl, aber auch z. B. 1-, 2- oder 3-Methylcyclopentylmethyl, 1-, 2-, 3- oder 4-Methylcyclohexylmethyl.

Bicycloalkyl bedeutet vorzugsweise 1- oder 2-Dekalyl, 2-Bicyclo[2,2,1]heptyl oder 6,6-Dimethyl-2-bicyclo[3,1,1]heptyl.

Tricycloalkyl bedeutet vorzugsweise 2-Adamantyl.

Ar bedeutet vorzugsweise Phenyl, ferner o-, m- oder p-Tolyl, o-, m- oder p-Ethylphenyl, o-, m- oder p-Methoxyphenyl, o-, m- oder p-Fluorphenyl, o-, m- oder p-Chlorphenyl, o-, m- oder p-Bromphenyl, o-, m- oder p-Jodphenyl, o-, m- oder p-Trifluormethylphenyl, 2,3-, 2,4-, 2,5-, 2,6-, 3,4- oder 3,5-Dimethoxyphenyl, 3,4,5-Trimethoxyphenyl, o-, m- oder p-Aminophenyl, 1- oder 2-Naphthyl.

$R^1$ bedeutet vorzugsweise A, insbesondere Methyl, Ethyl, Propyl, Isopropyl, Butyl, Isobutyl oder tert.-Butyl, weiterhin vorzugsweise Cyclopropyl, Cyclopentyl, Cyclohexyl, Phenyl oder Benzyl.

$R^2$, $R^5$ und $R^6$ bedeuten vorzugsweise H oder Methyl, ferner Ethyl, Propyl, Isopropyl, Butyl oder Isobutyl.

$R^3$ bedeutet vorzugsweise A, insbesondere Methyl, Ethyl, Propyl, Isopropyl, Isobutyl, sek.-Butyl, Pentyl, Isopentyl (3-Methylbutyl) oder 2-Methylbutyl; Cyclohexylmethyl, 2-Cyclohexylethyl; Benzyl, p-Chlorbenzyl; Bicyclo[2,2,1]heptyl-2-methyl, 6,6-Dimethylbicyclo[3,1,1]heptyl-2-methyl. Besonders bevorzugte Reste $R^3$ sind Isobutyl, Benzyl und Cyclohexylmethyl.

m, p, r und t sind vorzugsweise 0, 1 oder 2; n ist vorzugsweise 1.

X bedeutet vorzugsweise H, POA, Alkoxycarbonyl wie BOC, CBZ, Alkanoyl wie Acetyl, Propionyl, Butyryl oder Isobutyryl, Cycloalkylcarbonyl wie Cyclopentylcarbonyl oder Cyclohexylcarbonyl, Aroyl wie Benzoyl, Arylalkanoyl wie Phenylacetyl, 2- oder 3-Phenylpropionyl, 4-Phenylbutyryl, 2-Benzyl-4-phenylbutyryl, 2- oder 3-o-, -m- oder -p-Fluorphenylpropionyl,2- oder 3-o-, -m- oder -p-Chlorphenylpropionyl, Cycloalkylalkanoyl wie Cyclohexylacetyl, 2- oder 3-Cyclohexylpropionyl. Besonders bevorzugte Reste X sind H, BOC, CBZ, 4-Phenylbutyryl und 2-Benzyl-4-phenylbutyryl.

Z bedeutet O (= Valenz) oder 1, vorzugsweise 2, 3 oder 4 peptidartig miteinander verbundene Aminosäurereste, insbesondere die Gruppen His, Phe-His, Pro-Phe-His oder His-Pro-Phe-His, ferner bevorzugt die Gruppen Abu, Ada,

Asn, Bia, Gln, N-(im)-Alkyl-His, Leu, Nle, Phe, Trp, Tyr, Abu-His, Ada-His, Ala-His, Ala-Phe, Arg-His, Asn-His, Bia-His, Dab-His, Gly-His, His-His, Ile-His, Leu-His, tert.-Leu-His, Lys-His, Met-His, Nbg-His, Nle-His, (N-Me-His)-His, (N-Me-Phe)-His, Orn-His, Phe-Abu, Phe-Ada, Phe-Ala, Phe-Arg, Phe-Asn, Phe-Bia, Phe-Dab, Phe-Gln, Phe-Gly, Phe-(N-im-Alkyl-His), Phe-Ile, Phe-Leu, Phe-tert.-Leu, Phe-Lys, Phe-Met, Phe-Nbg, Phe-Nle, Phe-(N-Me-His), Phe-(N-Me-Phe), Phe-Orn, Phe-Phe, Phe-Pro, Phe-Ser, Phe-Thr, Phe-Tic, Phe-Trp, Phe-Tyr, Phe-Val, Pro-His, Ser-His, Thr-His, Tic-His, Trp-His, Tyr-His, Val-His, Ada-Phe-His, Pro-Ala-His, Pro-Ala-Phe, Pro-Phe-Ala, Pro-Phe-Phe, His-Pro-Ala-His, His-Pro-Ala-Phe, His-Pro-Phe-Ala, His-Pro-Phe-Phe, weiterhin Pro-Abu-His, Pro-Ada-His, Pro-Arg-His, Pro-Asn-His, Pro-Bia-His, Pro-Dab-His, Pro-Gly-His, Pro-His-His, Pro-Ile-His, Pro-Leu-His, Pro-tert.-Leu-His, Pro-Lys-His, Pro-Met-His, Pro-Nbg-His, Pro-Nle-His, Pro-(N-Me-His)-His, Pro-(N-Me-Phe)-His, Pro-Orn-His, Pro-Phe-Abu, Pro-Phe-Ada, Pro-Phe-Arg, Pro-Phe-Asn, Pro-Phe-Bia, Pro-Phe-Dab, Pro-Phe-Gln, Pro-Phe-Gly, Pro-Phe-(N-im-Alkyl-His), Pro-Phe-Ile, Pro-Phe-Leu, Pro-Phe-tert.-Leu, Pro-Phe-Lys, Pro-Phe-Met, Pro-Phe-Nbg, Pro-Phe-Nle, Pro-Phe-(N-Me-His), Pro-Phe-(N-Me-Phe), Pro-Phe-Orn, Pro-Phe-Pro, Pro-Phe-Ser, Pro-Phe-Thr, Pro-Phe-Tic, Pro-Phe-Trp, Pro-Phe-Tyr, Pro-Phe-Val, Pro-Pro-His, Pro-Ser-His, Pro-Thr-His, Pro-Tic-His, Pro-Trp-His, Pro-Tyr-His, Pro-Val-His, His-Pro-Abu-His, His-Pro-Ada-His, His-Pro-Arg-His, His-Pro-Asn-His, His-Pro-Bia-His, His-Pro-Dab-His, His-Pro-Gly-His, His-Pro-His-His, His-Pro-Ile-His, His-Pro-Leu-His, His-Pro-tert.-Leu-His, His-Pro-Lys-His, His-Pro-Met-His, His-Pro-Nbg-His, His-Pro-Nle-His, His-Pro-(N-Me-His)-His, His-Pro-(N-Me-Phe)-His, His-Pro-Orn-His, His-Pro-Phe-Abu, His-Pro-Phe-Ada, His-Pro-Phe-Arg, His-Pro-Phe-Asn, His-Pro-

Phe-Bia, His-Pro-Phe-Dab, His-Pro-Phe-Gln, His-Pro-Phe-Gly, His-Pro-Phe(N-im-Alkyl-His), His-Pro-Phe-Ile, His-Pro-Phe-Leu, His-Pro-Phe-tert.-Leu, His-Pro-Phe-Lys, His-Pro-Phe-Met, His-Pro-Phe-Nbg, His-Pro-Phe-Nle, His-Pro-Phe-(N-Me-His), His-Pro-Phe-(N-Me-Phe), His-Pro-Phe-Orn, His-Pro-Phe-Pro, His-Pro-Phe-Ser, His-Pro-Phe-Thr, His-Pro-Phe-Tic, His-Pro-Phe-Trp, His-Pro-Phe-Tyr, His-Pro-Phe-Val, His-Pro-Pro-His, His-Pro-Ser-His, His-Pro-Thr-His, His-Pro-Tic-His, His-Pro-Trp-His, His-Pro-Tyr-His, His-Pro-Val-His.

E ist vorzugsweise abwesend oder bedeutet vorzugsweise Ile, ferner vorzugsweise Leu, weiterhin Abu, Ala, Met, Nle oder Val.

Die Gruppe W bedeutet $-NR^2-CHR^3-CHOH-(CHR^5)_n-CO-$, vorzugsweise $-NM-CHR^3-CHOH-CH_2-CO-$, insbesondere $-NH-CH(Isobutyl)-CHOH-CH_2-CO-$ ("Sta"; abgeleitet von Statin), $-NH-CH(Benzyl)-CHOH-CH_2-CO-$ ("AHPP"; abgeleitet von 4-Amino-3-hydroxy-5-phenylpentansäure), $-NH-CH(Cyclohexylmethyl)-CHOH-CH_2-CO-$ ("AHCP", abgeleitet von 4-Amino-3-hydroxy-5-cyclohexylpentansäure) oder $-NH-CH(CH_2CH_2-Cyclohexyl)-CHOH-CH_2-CO-$ ("AHCH"; abgeleitet von 4-Amino-3-hydroxy-6-cyclohexyl-hexansäure) oder $-NR^2-CHR^3-CH(NH_2)-(CHR^5)_n-CO$, vorzugsweise $-NH-CHR^3-CH(NH_2)-CH_2-CO-$, insbesondere $-NH-CH-(Isobutyl)-CH(NH_2)-CH_2-CO-$ ("DAMH"; abgeleitet von 3,4-Diamino-6-methylheptansäure), $-NH-CH(Benzyl)-CH(NH_2)-CH_2-CO-$ ("DAPP"; abgeleitet von 3,4-Diamino-5-phenylpentansäure), $-NH-CH(Cyclohexylmethyl)-CH(NH_2)-CH_2-CO-$ ("DACP"; abgeleitet von 3,4-Diamino-5-cyclohexylpentansäure) oder $-NH-CH-(CH_2CH_2Cyclohexyl)-CH(NH_2)-CH_2-CO-$ ("DACH"; abgeleitet von 3,4-Diamino-6-cyclohexyl-hexansäure).

Die Gruppe W besitzt mindestens zwei chirale Zentren. Sie kann daher in verschiedenen - optisch-inaktiven oder optisch-aktiven - Formen vorkommen. Die Formel I umschließt alle diese Formen. Falls W $-NH-CHR^3-CHR^4-CH_2-CO-$ bedeutet, sind die 3S-Hydroxy-4S-amino-Enantiomeren bzw. die 3S,4S,-Diamino-Enantiomeren bevorzugt. Wenn bei der Bezeichnung von Einzelsubstanzen nichts anderes angegeben ist, beziehen sich die Abkürzungen Sta, AHPP, AHCP, AHCH, DAMH, DAPP, DACP und DACH immer auf diese 3S,4S-Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis If ausgedrückt werden, die der Formel I entsprechen, worin jedoch

in Ia

| | |
|---|---|
| X | H, POA, BOC, 4-Phenylbutyryl, 2-Benzyl-4-phenylbutyryl oder CBZ, |
| Z | abwesend, His, Phe-His, Pro-Phe-His oder His-Pro-Phe-His, |
| W | (a) $-NH-CHR^3-CHOH-CH_2-CO-$ oder (b) $-NH-CHR^3-CH(NH_2)-CH_2CO-$, |
| E | abwesend, Ile oder Leu, |
| $R^2$ | H, |
| $R^3$ | Isobutyl, Benzyl oder Cyclohexylmethyl, |
| | Q NH und |
| | $(CHR^5)_n$ $CH_2$ |
| | bedeuten; |

In Ib

| | |
|---|---|
| X | H oder BOC, |
| Z | Phe-His, |
| W | Sta, DAMH, DACP oder DAPP, |
| E | abwesend, Ile oder Leu, |
| $R^2$ | H, |
| $R^3$ | iso-$C_4H_9$, |
| | $(CHR^5)_n$ $CH_2$ und |
| | Q NH bedeuten; |

in Ic

X BOC bedeutet und

Z, W, E, $R^2$, $R^2$, $(CHR^5)_n$ und Q die bei Formel Ib angegebene Bedeutung haben.

Gegenstand der Erfindung ist ferner ein Verfahren zur Herstellung eines Peptids der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt

oder daß man eine Verbindung, die der Formel I entspricht, aber an Stelle von H-Atomen eine oder mehrere zusätzliche hydrogenolytisch abspaltbare Gruppen und/oder C-C- und/oder C-N- und/oder C-0-Bindungen enthält, reduziert

oder daß man ein Aminoketosäurederivat, das der Formel I entspricht, jedoch an Stelle einer $CH(NH_2)$-Gruppe eine C0-Gruppe enthält, reduktiv aminiert

oder daß man eine Carbonsäure der Formel II

$X\text{-}G^1\text{-}OH$ II

worin

$G^1$

(a) $Z^1$,

(b) Z,

(c) Z-W,

(d) Z-W-E bedeutet

mit einem Amin der Formel III

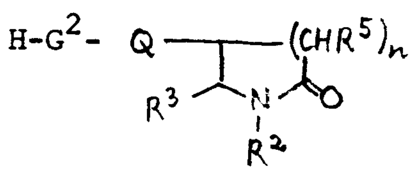

III

worin

$G^2$

(a) $Z^2$-W-E,

(b) W-E,

(c) E,

(d) fehlt und

$Z^1 + Z^2$ zusammen Z bedeuten

umsetzt,

und daß man gegebenenfalls in einer Verbindung der Formel I eine funktionell abgewandelte Amino- und/oder Hydroxygruppe durch Behandeln mit solvolysierenden oder hydrogenolysierenden Mitteln in Freiheit setzt und/oder eine Verbindung der Formel I durch Behandeln mit einer Säure oder Base in eines ihrer Salze überführt.

Die Verbindungen der Formel I und auch die Ausgangsstoffe zu ihrer Herstellung werden im übrigen nach an sich bekannten Methoden hergestellt, wie sie in der Literatur (z. B. in den Standardwerken wie Houben-Weyl, Methoden der Organischen Chemie, Georg-Thieme-Verlag, Stuttgart; ferner EP-A-45665, EP-A-77028, EP-A-77029, EP-A-81783) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Vorzugsweise werden die Verbindungen der Formel I erhalten, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die an Stelle einer oder mehrerer freier Amino- und/oder Hydroxygruppen entsprechende geschützte Amino- und/oder Hydroxygruppen enthalten, vorzugsweise solche, die an Stelle eines H-Atoms, das mit einem N-Atom verbunden ist, eine Aminoschutzgruppe tragen, insbesondere diejenigen der Formel IV

$X\text{-}Z\text{-}W^1\text{-}E\text{-}$ IV

worin

W[1] $-NR^2-CHR^3-CH(NHR^7)-(CHR^5)_n-CO-$, und
$R^7$ eine Aminoschutzgruppe,

bedeuten.

Ferner sind Ausgangsstoffe bevorzugt, die an Stelle des H-Atoms einer Hydroxygruppe eine Hydroxyschutzgruppe tragen.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls duchgeführt worden ist. Typisch für solche Gruppen sind insbesondere unsubstituierte oder substituierte Acylgruppen, ferner unsubstituierte oder substituierte Aryl(z. B. 2,4-Dinitrophenyl) oder Aralkylgruppen (z. B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxycarbonyl, BOC, 2-Jodethoxycarbonyl; Aralkyloxycarbonyl wie CBZ ("Carbobenzoxy"), 4-Methoxybenzyloxycarbonyl, FMOC. Bevorzugte Aminoschutzgruppen sind CBZ, FMOC, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden der Aminosäure- und Peptidsynthese hergestellt werden, wie sie z. B. in den genannten Standardwerken und Patentanmeldungen beschrieben sind.

Das In-Freiheit-Setzen der Verbindungen der Formel I aus ihren funktionellen Derivaten gelingt - je nach der benutzten Schutzgruppe - z. B. mit starken Säuren, zweckmäßig mit Trifluoressigsäure oder Perchlorsäure aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie Dimethylformamid (DMF), halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9 : 1. Die Reaktionstemperaturen für die Spaltung liegen zweckmäßig zwischen etwa 0 und etwa 50°, vorzugsweise arbeitet man zwischen 15 und 30° (Raumtemperatur).

Die BOC-Gruppe kann z. B. bevorzugt mit 40 %iger Trifluoressigsäure in Methylenchlorid oder mit etwa 3 bis 5 n HCl in Dioxan bei 15-30° abgespalten werden, die FMOC-Gruppe mit einer etwa 5- bis 20%igen Lösung von Dimethylamin, Diethylamin oder Piperidin in DMF bei 15-30°. Eine Abspaltung der DNP-Gruppe gelingt z. B. auch mit einer etwa 3- bis 10%igen Lösung von 2-Mercaptoethanol in DMF/Wasser bei 15-30°. Nach der "Merrifield"-Methode werden Verbindungen der Formel I (Y = OH) zweckmäßig mit Trifluoressigsäure von dem polymeren Träger abgespalten.

Hydrogenolytisch entfernbare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5- bis 10%igem Pd-C in Methanol bei 20-30°.

Die Verbindungen der Formel I sind auch erhältlich durch Reduktion entsprechender Verbindungen, die an Stelle von H-Atomen eine oder mehrere zusätzliche hydrogenolytisch abspaltbare Gruppen und/oder C-C- und/oder C-N- und/oder C-O-Bindungen enthalten.

So können z. B. Ketoverbindungen
zu Verbindungen der Formel I reduziert werden, beispielsweise mit einem komplexen Metallhydrid wie $NaBH_4$, das nicht gleichzeitig die Peptid-Carbonylgruppen reduziert, in einem inerten Lösungsmittel wie Methanol bei Temperaturen zwischen etwa -10 und +30°.

Die Verbindungen der Formel I können auch durch reduktive Aminierung von Aminoketosäurederivaten hergestellt werden.

Man kann ein- oder mehrstufig reduktiv aminieren. So kann man das Aminoketosäurederivat mit Ammoniumsalzen, z. B. Ammoniumacetat, und $NaCNBH_3$ behandeln, vorzugsweise in einem inerten Lösungsmittel, z. B. einem Alkohol wie Methanol bei Temperaturen zwischen etwa 0 und 50°, insbesondere zwischen 15 und 30°.

Weiterhin ist es möglich, das Keton zunächst mit Hydroxylamin in üblicher Weise in das Oxim zu überführen urd dieses, z. B. durch katalytische Hydrierung an Raney-Nickel, zum Amin zu reduzieren.

Verbindungen der Formel I, insbesondere solche, in denen W $-NR^2-CHR^3-CHOH-(CHR^5)_n-CO-$bedeutet, können auch durch direkte Peptidsynthese aus einer Carbonsäure- und einer Aminokomponente erhalten werden. Als Carbonsäurekomponenten eignen sich z. B. solche der Teilformeln X-Z-OH, X-Z-W-OH oder X-Z-W-E-OH, aLS Aminokomponenten solche der Teilformeln H-W-E-W', H-E-W' oder H-W', worin W' für die Gruppe

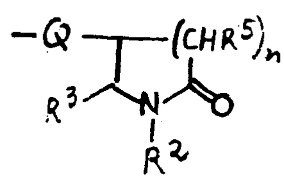

steht. Die Peptidbindung kann aber auch innerhalb der Gruppe Z geknüpft werden; dabei wird eine Carbonsäure der Formel $X-Z^1-OH$ mit einem Aminopeptid der Formel $H-Z^2-W-E-W'$ umgesetzt, wobei $Z^1 + Z^2 = Z$ ist. Dabei arbeitet man zweckmäßig nach üblichen Methoden der Peptid-Synthese, wie sie z. B. in Houben-Weyl, 1.c., Band, 15/II, Seiten 1 bis 806 (1974) beschrieben sind.

Die Reaktion gelingt vorzugsweise in Gegenwart eines Dehydratisierungsmittels, z. B. eines Carbodiimids wie DCCI oder Dimethylaminopropylethyl-carbodiimid, ferner Propanphosphonsäureanhydrid (vgl. Angew.Chem. 92, 129 (1980)), Diphenylphosphorylazid oder 2-Ethoxy-N-ethoxycarbonyl-1,2-dihydrochinolin, in einem inerten Lösungsmittel, z. B. einem halogenierten Kohlenwasserstoff wie Dichlormethan, einem Ether wie Tetrahydrofuran oder Dioxan, einem Amid wie Dimethylformamid (DMF) oder Dimethylacetamid, einem Nitril wie Acetonitril, bei Temperaturen zwischen etwa -10 und 40, vorzugsweise zwischen 0 und 30°.

An Stelle von II bzw. III können auch geeignete reaktionsfähige Derivate dieser Stoffe in die Reaktion eingesetzt werden, z. B. solche, in denen reaktive Gruppen intermediär durch Schutzgruppen blockiert sind. Die Aminosäurederivate III können z. B. in Form ihrer aktivierten Ester verwendet werden, die zweckmäßig in situ gebildet werden, z. B. durch Zusatz von 1-Hydroxybenztriazol oder N-Hydroxysuccinimid.

Die Ausgangsstoffe der Formel II und III sind großenteils bekannt. Sofern sie nicht bekannt sind, können sie nach bekannten Methoden, z. B. den oben angegebenen Methoden der Peptidsynthese und der Abspaltung von Schutzgruppen, hergestellt werden.

Gewünschtenfalls kann in einer Verbindung der Formel I eine funktionell abgewandelte Amino- und/oder Hydroxygruppe durch Solvolyse oder Hydrogenolyse nach einer der oben beschriebenen Methoden in Freiheit gesetzt werden.

So kann insbesondere eine Verbindung der Formel I, worin X von H verschieden ist, in eine Verbindung der Formel I (X = H) umgewandelt werden, zweckmäßig durch Hydrogenolyse, falls X = CBZ ist, sonst durch selektive Solvolyse. Falls X = BOC ist, kann man z. B. mit HCl in Dioxan bei Raumtemperatur die

BOC-Gruppe abspalten.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z. B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und disulfonsäuren, Laurylschwefelsäure. Salze mit physiologisch nicht unbedenklichen Säuren, z. B. Pikrate, können zur Isolierung und/oder Aufreinigung der Verbindungen der Formel I verwendet werden.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z. B. rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin. Zur rektalen Anwendung dienen Suppositorien, zur parenteralen Applikation Lösungen, vorzugsweise ölige oder wässerige Lösungen, ferner Suspensionen, Emulsionen oder Implantate. Für die Applikation als Inhalatians-Spray können Sprays verwendet werden, die den Wirkstoff entweder gelöst oder suspendiert in einem Treibgasgemisch (z. B. Fluorchlor-kohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt worden. Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z. B. ein oder mehrere Vitamine.

Die erfindungsgemäßen Substanzen werden in der Regel in Analogie zu anderen bekannten, im Handel befindlichen Peptiden, insbesondere aber in Analogie zu den in der EP-A-77028 beschriebenen Verbindungen verabreicht, vorzugsweise in Dosierungen zwischen etwa 100 mg und 30 g, insbesondere zwischen 500 mg und 5 g pro Dosierungseinheit. Die tägliche Dosierung liegt vorzugsweise zwischen etwa 2 und 600 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, extrahiert mit Ether oder Dichlormethan, trennt ab, trocknet die organische Phase über Natriumsulfat, filtriert, dampft ein und reinigt durch Chromatographie an Kieselgel und/oder Kristallisation.

Beispiel 1

Man löst 1 g 3R-CBZ-amino-4S-[BOC-Phe-(imi-BOM-His)-Sta-Leu-amino]-5-phenylpentansäuremethylester ["BOC-Phe-(imi-BOM-His)-Sta-Leu-(3R-CBZ-DAPP)-OMe"; erhältlich durch Reaktion von BOC-DAPP-OMe mit Benzyloxycarbonylchlorid zu 3R-CBZ-amino-4S-BOC-amino-5-phenylpentansäuremethylester (F. 111-112°), Hydrolyse zu 3R-CBZ-amino-4S-amino-5-phenylpentansäuremethylester ("3R-CBZ-DAPP-OMe"), Umsetzung mit BOC-Leu-OH zu 3R-CBZ-amino-4S-BOC-Leu-amino-5-phenylpentansäuremethylester, Abspaltung der BOC-Gruppe mit HCl/Dioxan und Reaktion mit BOC-Phe-(imi-BOM-His)-Sta-OH/DCCI/HOBt] in 10 ml Methanol, hydriert an 0,5 g 10%ig. Pd-C bei 20 ° und 1 bar 3 Std. lang, filtriert, dampft ein und erhält BOC-Phe-His-Sta-Leu-(3R,4S)-DAPP-OMe, F. 203°. (Diese Verbindung wird nicht

beansprucht).

Beispiel 2

Analog Beispiel 1 erhält man durch Hydrogenolyse von 4R-[BOC-Phe-(imi-BOM-His)-Sta-Ile-amino]-5S-isobutyl-pyrrolidon[F. 120-123° (Zers.); erhältlich durch Cyclisierung von 3S-CBZ-amino-4R-BOC-amino-6-methylpentansäuremethylester-hydrochlorid mit 1 n Natronlauge bei 20° zu 4R-CBZ-amino-5S-isobutyl-pyrrolidon (F. 119°), Hydrogenolyse zu 4R-Amino-5S-isobutyl-pyrrolidon (Öl; Rf 0,13 an Kieselgel, Dichlormethan/Methanol 9:1), Kondensation mit BOC-Ile-OH/DCCI/HOBt zu 4R-BOC-Ile-amino-5S-isobutyl-pyrrolidon, Abspaltung der BOC-Gruppe mit HCl/Dioxan und Kondensation mit BOC-Phe-(imi-BOM-His)-Sta-OH (F. 156-158°)] das 4R-(BOC-Phe-His-Sta-Ile-amino)-5S-isobutyl-pyrrolidon, F. 105° (Zers.).

Analog erhält man durch Hydrogenolyse der entsprechenden (imi-BOM-His)-Derivate bzw. CBZ-Derivate:

4S-(BOC-Phe-His-Sta-Ile-amino)-5S-isobutyl-pyrrolidon
4R-(BOC-Phe-His-Sta-amino)-5S-isobutyl-pyrrolidon, F. 122-123°
4S-(BOC-Phe-His-Sta-amino)-5S-isobutyl-pyrrolidon
4S-(BOC-Phe-His-AHPP-amino)-5S-isobutyl-pyrrolidon
4S-(BOC-Phe-His-AHCP-amino)-5S-isobutyl-pyrrolidon, F. 195-196°
4S-(BOC-Phe-His-AHCH-amino)-5S-isobutyl-pyrrolidon
4S-(BOC-Phe-His-DAMH-amino)-5S-isobutyl-pyrrolidon
4S-(BOC-Phe-His-DAPP-amino)-5S-isobutyl-pyrrolidon
4S-(BOC-Phe-His-DACP-amino)-5S-isobutyl-pyrrolidon
4S-(BOC-Phe-His-DACH-amino)-5S-isobutyl-pyrrolidon, F. 205-208°
4S-(BOC-Phe-His-AHPP-Ile-amino)-5S-isobutyl-pyrrolidon
4S-(BOC-Phe-His-AHCP-Ile-amino)-5S-isobutyl-pyrrolidon
4S-(BOC-Phe-His-AHCH-Ile-amino)-5S-isobutyl-pyrrolidon
4S-(BOC-Phe-His-DAMH-Ile-amino)-5S-isobutyl-pyrrolidon
4S-(BOC-Phe-His-DAPP-Ile-amino)-5S-isobutyl-pyrrolidon
4S-(BOC-Phe-His-DACP-Ile-amino)-5S-isobutyl-pyrrolidon
4S-(BOC-Phe-His-DACH-Ile-amino)-5S-isobutyl-pyrrolidon
4S-(BOC-Phe-Abu-AHCP-amino)-5S-isobutyl-pyrrolidon
4S-(BOC-Phe-His-AHCP-amino)-5S-isobutyl-pyrrolidon
4S-(BOC-Phe-Leu-AHCP-amino)-5S-isobutyl-pyrrolidon
4S-(BOC-Phe-Nle-AHCP-amino)-5S-isobutyl-pyrrolidon
4S-[BOC-(N-Me-Phe)-His-AHCP-amino]-5S-isobutyl-pyrrolidon
4S-[(3-Phenylpropionyl)-(N-Me-Phe)-His-AHCP-amino]-5S-isobutyl-pyrrolidon
4S-[(2-Benzyl-4-phenylbutyryl)-(N-Me-Phe)-His-AHCP-amino]-5S-isobutyl-pyrrolidon
4S-(POA-His-Sta-Ile-amino)-5S-isobutyl-pyrrolidon
4S-[(3-Phenylpropionyl)-His-Sta-Ile-amino]-5S-isobutyl-pyrrolidon
4S-[(4-Phenylbutyryl)-His-Sta-Ile-amino]-5S-isobutyl-pyrrolidon
4S-[(2-Benzyl-3-phenyl-propionyl)-His-Sta-Ile-amino]-5S-isobutyl-pyrrolidon
4S-[(2-Benzyl-4-phenylbutyryl)-His-Sta-Ile-amino]-5S-isobutyl-pyrrolidon
4S-[(2-Benzyl-4-phenylbutyryl)-His-AHPP-Ile-amino]-5S-isobutyl-pyrrolidon
4S-[(2-Benzyl-4-phenylbutyryl)-His-AHCP-Ile-amino]-5S-isobutyl-pyrrolidon
4S-[(2-Benzyl-4-phenylbutyryl)-His-AHCH-Ile-amino]-5S-isobutyl-pyrrolidon
4S-[(2-Benzyl-4-phenylbutyryl)-His-DAMH-Ile-amino]-5S-isobutyl-pyrrolidon
4S-[(2-Benzyl-4-phenylbutyryl)-His-DAPP-Ile-amino]-5S-isobutyl-pyrrolidon
4S-[(2-Benzyl-4-phenylbutyryl)-His-DACP-Ile-amino]-5S-isobutyl-pyrrolidon
4S-[(2-Benzyl-4-phenylbutyryl)-His-DACH-Ile-amino]-5S-isobutyl-pyrrolidon
4S-(BOC-Phe-His-Sta-Abu-amino)-5S-isobutyl-pyrrolidon
4S-(BOC-Phe-His-Sta-Leu-amino)-5S-isobutyl-pyrrolidon
4S-(BOC-Phe-His-Sta-Met-amino)-5S-isobutyl-pyrrolidon
4S-(BOC-Phe-His-Sta-Nle-amino)-5S-isobutyl-pyrrolidon
4S-(BOC-Phe-His-Sta-Val-amino)-5S-isobutyl-pyrrolidon
4S-[BOC-(N-Me-Phe)-His-Sta-Ile-amino]-5S-isobutyl-pyrrolidon
4S-(BOC-Phe-Dab-Sta-Ile-amino)-5S-isobutyl-pyrrolidon
4S-(BOC-Phe-Lys-Sta-Ile-amino)-5S-isobutyl-pyrrolidon
4S-(BOC-Phe-Orn-Sta-Ile-amino)-5S-isobutyl-pyrrolidon

4S-(BOC-Trp-His-Sta-Ile-amino)-5S-isobutyl-pyrrolidon
4S-(BOC-Tyr-His-Sta-Ile-amino)-5S-isobutyl-pyrrolidon
sowie die 4R,5S-Epimeren dieser Verbindungen.

Beispiel 3

Eine Lösung von 1,92 g 4R-Amino-5S-isobutyl-pyrrolidon-hydrochlorid in 50 ml Dichlormethan wird mit 1,01 g N-Methylmorpholin versetzt. Man gibt 5,2 g BOC-Phe-Nle-Sta-OH, 1,35 g HOBt und eine Lösung von 2,06 g DCCI in 50 ml Dichlormethan hinzu, rührt 14 Std. bei 4°, filtriert den ausgefallenen Dicyclohexylharnstoff ab und dampft das Filtrat ein. Man nimmt in Ethylacetat auf, wäscht mit 1 n HCl und 1 n Natronlauge und trocknet über Magnesiumsulfat. Nach chromatographischer Reinigung an Kieselgel mit Dichlormethan/Methanol erbält man 4R-(BOC-Phe-Nle-Sta-amino)-5S-isobutyl-pyrrolidon, F. 131°.

Analog erhält man mit den entsprechenden Peptid-carbonsäuren:
4S-(BOC-Phe-Abu-Sta-Ile-amino)-5S-isobutyl-pyrrolidon
4S-(BOC-Phe-Asn-Sta-Ile-amino)-5S-isobutyl-pyrrolidon
4S-(BOC-Phe-Gln-Sta-Ile-amino)-5S-isobutyl-pyrrolidon
4S-(BOC-Phe-Leu-Sta-Ile-amino)-5S-isobutyl-pyrrolidon
4S-(BOC-Phe-Nle-Sta-Ile-amino)-5S-isobutyl-pyrrolidon
4S-[BOC-Phe-(N-imi-Methyl-His)-Sta-Ile-amino]-5S-isobutyl-pyrrolidon.

Beispiel 4

Analog Beispiel 1 erhält man durch Hydrogenolyse der entsprechenden CBZ-Derivate oder der entsprechenden CBZ-imi-BOM-His-Derivate oder der entsprechenden imi-BOM-His-Derivate:
4S-(BOC-Phe-His-Sta-amino)-5S-isobutyl-pyrrolidon
4S-(BOC-Phe-His-AHPP-amino)-5S-isobutyl-pyrrolidon
4S-(BOC-Phe-His-AHCP-amino)-5S-isobutyl-pyrrolidon, F. 195-196°
4S-(BOC-Phe-His-AHCH-amino)-5S-isobutyl-pyrrolidon
4S-(BOC-Phe-His-DAMH-amino)-5S-isobutyl-pyrrolidon
4S-(BOC-Phe-His-DAPP-amino)-5S-isobutyl-pyrrolidon
4S-(BOC-Phe-His-DACP-amino)-5S-isobutyl-pyrrolidon
4S-(BOC-Phe-His-DACH-amino)-5S-isobutyl-pyrrolidon
4R-(BOC-Phe-His-Sta-amino)-5S-benzyl-pyrrolidon, F. 133-135°
4R-(BOC-Phe-His-Sta-amino)-5S-isobutyl-pyrrolidon
4R-(BOC-Phe-His-AHPP-amino)-5S-isobutyl-pyrrolidon
4R-(BOC-Phe-His-AHCP-amino)-5S-isobutyl-pyrrolidon, F. 145-148°
4R-(BOC-Phe-His-AHCH-amino)-5S-isobutyl-pyrrolidon
4R-(BOC-Phe-His-DAMH-amino)-5S-isobutyl-pyrrolidon
4R-(BOC-Phe-His-DAPP-amino)-5S-isobutyl-pyrrolidon
4R-(BOC-Phe-His-DACP-amino)-5S-isobutyl-pyrrolidon
4R-(BOC-Phe-His-DACH-amino)-5S-isobutyl-pyrrolidon
4R-[(2-Benzyl-4-phenylbutyryl)-His-AHCP-amino]-5S-isobutyl-pyrrolidon
4S-[(2-Benzyl-4-phenylbutyryl)-His-AHCP-amino]-5S-isobutyl-pyrrolidon, F. 147-149°
4S-[(4-Phenylbutyryl)-His-AHCP-amino]-5S-isobutyl-pyrrolidon, F. 223° (Zers.)
4R-(BOC-Phe-His-AHCP-Ile-amino)-5S-isobutyl-pyrrolidon, F. 140-142°
4S-[(4,4-Diphenylbutyryl)-His-AHCP-amino]-5S-isobutyl-pyrrolidon, F. 123-125°
4S-[(3,3-Diphenylpropionyl)-His-AHCP-amino]-5S-isobutyl-pyrrolidon,F. 221-224°
4S-(POA-His-AHCP-amino)-5S-isobutyl-pyrrolidon, F. 110° (Zers.)
4S-(Benzoyl-His-AHCP-amino)-5S-isobutyl-pyrrolidon, F. 238° (Zers.)
4S-[2-(2-Phenylethyl)-4-phenylbutyryl-His-AHCP-amino]-5S-isobutyl-pyrrolidon, F. 166-168°
4S-[(2-Benzyl-3-phenylpropionyl)-His-AHCP-amino]-5S-isobutyl-pyrrolidon, F. 154-157°
4R-[BOC-(N-Me-Phe)-His-AHCP-amino]-5S-isobutyl-pyrrolidon, F. 210-215°
4R-(BOC-Tic-His-AHCP-amino)-5S-isobutyl-pyrrolidon, F. 165°
4R-(BOC-Phe-Orn-AHCP-amino)-5S-isobutyl-pyrrolidon
4R-(BOC-Phe-Lys-AHCP-amino)-5S-isobutyl-pyrrolidon
4R-(BOC-Phe-Dab-AHCP-amino)-5S-isobutyl-pyrrolidon
4R-(2-Benzyl-4-phenylbutyryl-Orn-AHCP-amino)-5S-isobutyl-pyrrolidon
4R-(2-Benzyl-4-phenylbutyryl-Lys-AHCP-amino)-5S-isobutyl-pyrrolidon

4R-(2-Benzyl-4-phenylbutyryl-Dab-AHCP-amino)-5S-isobutyl-pyrrolidon.

Beispiel 5

Analog Beispiel 3 erhält man:
4R-(BOC-Phe-Ala-AHCP-amino)-5S-isobutyl-pyrrolidon
4R-(2-Benzyl-4-phenylbutyryl-Ala-AHCP-amino)-5S-isobutyl-pyrrolidon.

**Patentansprüche**

**1.** Peptide der Formel I

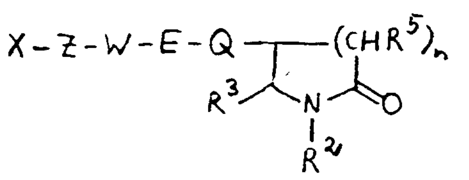

I

worin

| | |
|---|---|
| X | H, $R^1$-0-$C_mH_{2m}$-C0-, $R^1$-$C_mH_{2m}$-0-C0-, $R^1$-$C_mH_{2m}$-C0-, $R^1$-$S0_2$-, ($R^1$-$C_mH_{2m}$)-L($R^1$-$C_pH_{2p}$)-$C_rH_{2r}$-C0-, H-($NHCH_2CH_2$)$_m$-NH-$CH_2$C0- oder 9-Fluorenyl-$C_mH_{2m}$-0-C0, |
| Z | 0 bis 4 peptidartig miteinander verbundene Aminosäurereste ausgewählt aus der Gruppe bestehend aus Abu, Ada, Ala, Arg, Asn, Bia, Dab, Gln, Gly, His, N(im)-Alkyl-His, Ile, Leu, tert.-Leu, Lys, Met, Nbg, Nle, Orn, Phe, Pro, Ser, Thr, Tic, Trp, Tyr und Val, |
| W | -$NR^2$-$CHR^3$-$CHR^4$-($CHR^5$)$_n$-C0-, |
| E | 0 bis 2 peptidartig miteinander verbundene Aminosäurereste ausgewählt aus der Gruppe bestehend aus Abu, Ala, Ile, Leu, Met, Nle und Val, |
| $R^1$ und $R^3$ | jeweils A, Ar, Ar-alkyl, unsubstituiertes oder ein- oder mehrfach durch Alkyl, Alkoxy und/oder Hal substituiertes Cycloalkyl mit 3-7 C-Atomen, Cycloalkyl-alkyl mit 4-11 C-Atomen, Bicycloalkyl oder Tricycloalkyl mit jeweils 7-14 C-Atomen, Bicycloalkyl-alkyl oder Tricycloalkyl-alkyl mit jeweils 8-18 C-Atomen, |
| $R^2$ und $R^5$ | jeweils H oder A, |
| $R^4$ | OH oder $NH_2$, |
| L | CH oder N, |
| m, p und r | jeweils 0, 1, 2, 3, 4, oder 5, |
| n | 1 oder 2, |
| Q | 0 oder NH, |
| Ar | unsubstituiertes oder ein- oder mehrfach durch A, A0, Hal, $CF_3$, 0H und/oder $NH_2$ substituiertes Phenyl oder unsubstituiertes Naphthyl, |
| Hal | F, Cl, Br oder J und |
| A | Alkyl mit 1-8 C-Atomen bedeuten, |

worin ferner an Stelle einer oder mehrerer -NH-C0-Gruppen auch eine oder mehrere -N(Alkyl)-C0-Gruppen stehen können,
wobei die Reste $R^2$, $R^3$ und $R^5$ sowie die Parameter n gleich oder verschieden sein können,
sowie deren Salze.

**2.** 4R-(BOC-Phe-His-AHCP-Ile-amino)-5S-isobutyl-pyrrolidon.

**3.** Verfahren zur Herstellung eines Peptids der Formel I nach Patentanspruch 1 sowie von seinen Salzen, dadurch gekennzeichnet, daß man es aus einem seiner funktionellen Derivate durch Behandeln mit

einem solvolysierenden oder hydrogenolysierenden Mittel in Freiheit setzt
oder daß man eine Verbindung, die der Formel I entspricht, aber an Stelle von H-Atomen eine oder mehrere zusätzliche hydrogenolytisch abspaltbare Gruppen und/oder C-C- und/oder C-N- und/oder C-0-Bindungen enthält, reduziert
oder daß man ein Aminoketosäurederivat, das der Formel I entspricht, jedoch an Stelle einer $CH(NH_2)$-Gruppe eine C0-Gruppe enthält, reduktiv aminiert
oder daß man eine Carbonsäure der Formel II

$X\text{-}G^1\text{-}OH$ II

worin
$G^1$
(a) $Z^1$,
(b) Z,
(c) Z-W,
(d) Z-W-E bedeutet
mit einem Amin der Formel III

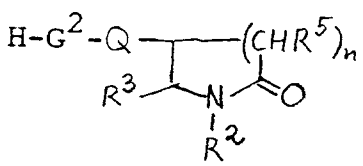

III

worin
$G^2$
(a) $Z^2$-W-E,
(b) W-E,
(c) E,
(d) fehlt und
$Z^1$ + $Z^2$ zusammen Z bedeuten
umsetzt,
und daß man gegebenenfalls in einer Verbindung der Formel I eine funktionell abgewandelte Amino- und/oder Hydroxygruppe durch Behandeln mit solvolysierenden oder hydrogenolysierenden Mitteln in Freiheit setzt und/oder eine Verbindung der Formel I durch Behandeln mit einer Säure oder Base in eines ihrer Salze überführt.

**4.** Verfabren zur Herstellung pharmazeutischer Zubereitungen, dadurch gekennzeichnet, daß man eine Verbindung der Formel I nach Patentanspruch I und/oder eines ihres physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

**5.** Pharmazeutische Zubereitung, gekennzeichnet durch einen Gehalt an mindestens einer Verbindung der Formel I nach Patentanspruch 1 und/oder einem ihrer physiologisch unbedenklichen Salze.

**6.** Verbindungen der Formel I nach Patentanspruch 1 und deren physiologisch unbedenklichen Salze zur Bekämpfung von Krankheiten.

**7.** Verwendung von Verbindungen der Formel I nach Patentanspruch 1 oder von deren physiologisch unbedenklichen Salzen zur Herstellung eines Arzneimittels zur Bekämpfung der reninabhängigen Hypertension oder des Hyperaldosteronismus.

## Claims

**1.** Peptides of the formula I

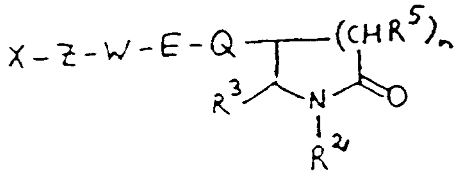

I

wherein

| | |
|---|---|
| X | is H, $R^1$-O-$C_mH_{2m}$-CO-, $R^1$-$C_mH_{2m}$-O-CO-, $R^1$-$C_mH_{2m}$-CO-, $R^1$-$SO_2$-, ($R^1$-$C_m$-$H_{2m}$)-L($R^1$-$C_pH_{2p}$)-$C_rH_{2r}$-CO-, H-($NHCH_2CH_2$)$_m$-NH-$CH_2$CO- or 9-fluorenyl-$C_mH_{2m}$-O-CO, |
| Z | is 0 to 4 amino acid radicals bonded together in peptide form and chosen from the group comprising |

Abu, Ada, Ala, Arg, Asn, Bia, Dab, Gln, Gly, His, N(im)-alkyl-His, Ile, Leu, tert.-Leu, Lys, Met, Nbg, Nle, Orn, Phe, Pro, Ser, Thr, Tic, Trp, Tyr and Val,

| | |
|---|---|
| W | is -$NR^2$-$CHR^3$-$CHR^4$-($CHR^5$)$_n$-CO-, |
| E | is 0 to 2 amino acid radicals bonded to one another in peptide form and chosen from the group comprising Abu, Ala, Ile, Leu, Met, Nle and Val, |
| $R^1$ and $R^3$ | are each A, Ar, Ar-alkyl, cycloalkyl which has 3-7 C atoms and is unsubstituted or mono- or poly-substituted by alkyl, alkoxy and/or Hal, cycloalkyl-alkyl with 4-11 C atoms, bicycloalkyl or tricycloalkyl with in each case 7-14 C atoms or bicycloalkyl-alkyl or tricycloalkyl-alkyl with in each case 8-18 C atoms, |
| $R^2$ and $R^5$ | are each H or A, |
| $R^4$ | is OH or $NH_2$, |
| L | is CH or N, |
| m, p and r | are each 0, 1, 2, 3, 4 or 5, |
| n | is 1 or 2, |
| Q | is O or NH, |
| Ar | is phenyl which is unsubstituted or mono- or poly-substituted by A, AO, Hal, $CF_3$, OH and/or $NH_2$, or is unsubstituted naphthyl, |
| Hal | is F, Cl, Br or I and |
| A | is alkyl with 1-8 C atoms, |

and, wherein, furthermore, one or more -NH-CO- groups can also be replaced by one or more -N(alkyl)-CO- groups, and wherein the radicals $R^2$, $R^3$ and $R^5$ and the parameters n can be identical or different, and salts thereof.

**2.** 4R-(BOC-Phe-His-AHCP-Ile-amino)-5S-isobutyl-pyrrolidone.

**3.** Process for the preparation of a peptide of the formula I according to Patent Claim 1 and of its salts, characterised in that it is liberated from one of its functional derivatives by treatment with a solvolysing or hydrogenolysing agent,
or in that a compound which corresponds to the formula I but contains one or more additional groups which can be split hydrogenolytically and/or C-C and/or C-N and/or C-O bonds instead of H atoms is reduced,
or in that an amino-keto acid derivative which corresponds to the formula I but contains a CO group instead of a $CH(NH_2)$ group is aminated reductively,
or in that a carboxylic acid of the formula II

$X$-$G^1$-OH II

wherein

G[1] is

(a) $Z^1$,
(b) Z,
(c) Z-W or
(d) Z-W-E

is reacted with an amino of the formula III

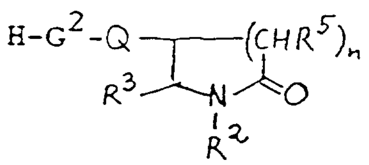

wherein

$G^2$ is

(a) $Z^2$-W-E,
(b) W-E,
(c) E or
(d) is absent and

$Z^1$ + $Z^2$ together are Z,

and in that, if appropriate, a functionally modified amino and/or hydroxyl group in a compound of the formula I is liberated by treatment with solvolysing or hydrogenolysing agents and/or a compound of the formula I is converted into one of its salts by treatment with an acid or base.

**4.** Process for the preparation of pharmaceutical formulations, characterised in that a compound of the formula I according to Patent Claim 1 and/or one of its physiologically acceptable salts are brought into a suitable dosage form together with at least one solid, liquid or semi-liquid excipient or auxiliary and, if appropriate, in combination with one or more other active compound(s).

**5.** Pharmaceutical formulation, characterised in that it contains at least one compound of the formula I according to Patent Claim 1 and/or one of its physiologically acceptable salts.

**6.** Compounds of the formula I according to Patent Claim 1 and physiologically acceptable salts thereof, for the treatment of diseases.

**7.** Use of compounds of the formula I according to Patent Claim 1 or of physiologically acceptable salts thereof, for the preparation of a medicament for combating renin-dependent hypertension or hyperaldosteronism.

**Revendications**

**1.** Peptides de formule I

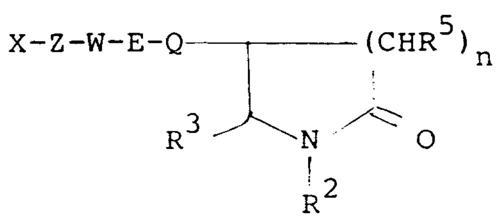

I

où

X représente H, $R^1$-O-$C_mH_{2m}$-CO-, $R^1$-$C_mH_{2m}$-O-CO-,$R^1$-$C_mH_{2m}$-CO-, $R^1$-$SO_2$-, ($R^1$-$C_mH_{2m}$)-L($R^1$-$C_pH_{2p}$)-$C_rH_{2r}$-CO-, H-($NHCH_2CH_2$)$_m$-NH-$CH_2$CO- ou 9-fluorényl-$C_mH_{2m}$-O-CO,

Z représente 0 à 4 restes d'acides aminés reliés entre eux à la façon des peptides,

choisis dans le groupe constitué par

Abu, Ada, Ala, Arg, Asn, Bia, Dab, Gln, Gly, His, N(im)-alkyl-His, Ile, Leu, tert-Leu, Lys, Met, Nbg, Nle, Orn, Phe, Pro, Ser, Thr, Tic, Trp, Tyr et Val,

| | |
|---|---|
| W | représente $-NR^2-CHR^3-CHR^4-(CHR^5)_n-CO-$, |
| E | 0 à 2 restes d'acides aminés reliés entre eux à la façon des peptides, choisis dans le groupe constitué par Abu, Ala, Ile, Leu, Met, Nle et Val, |
| $R^1$ et $R^3$ | représentent A, Ar, Ar-alkyle, un cycloalkyle ayant 3 à 7 atomes de C non substitué ou mono- ou polysubstitué par un alkyle, un alcoxy et/ou Hal, un cycloalkyle-alkyle ayant 4 à 11 atomes de C, un bicycloalkyle ou un tricycloalkyle ayant 7 à 14 atomes de C, un bicycloalkyle-alkyle ou un tricycloalkyle-alkyle ayant 8 à 18 atomes de C, |
| $R^2$ et $R^5$ | représentent H ou A, |
| $R^4$ | OH ou $NH_2$, |
| L | CH ou N |
| m, p et r | 0, 1, 2, 3, 4 ou 5, |
| m | 1 ou 2, |
| Q | O ou NH, |
| Ar | un phényle non substitué ou mono- ou polysubstitué par A, AO, Hal, $CF_3$, OH et/ou $NH_2$ ou un naphtyle non substitué, |
| Hal | F, Cl, Br ou I et |
| A | un alkyle ayant 1 à 8 atomes de C |

où, en outre, à la place d'un ou plusieurs groupes -NH-CO-, on peut également avoir un ou plusieurs groupes -N(alkyl)-CO-,
les restes $R^2$, $R^3$ et $R^5$ ainsi que le paramètre n pouvant être identiques ou différents,
ainsi que leurs sels.

**2.** 4R-(BOC-Phe-His-AHCP-Ile-amino)-5S-isobutyl-pyrrolidone.

**3.** Procédé pour la préparation d'un peptide de formule I selon la revendication 1 ainsi que de ses sels, caractérisé en ce qu'on libère ledit peptide de l'un de ses dérivés fonctionnels par traitement avec un agent solvolysant ou hydrogénolysant
ou en ce que l'on réduit un composé correspondant à la formule I, mais qui contient, à la place d'atomes de H un ou plusieurs groupes supplémentaires clivables par hydrogénolyse et/ou des liaisons C-C et/ou C-N et/ou C-O
ou en ce que l'on amine par voie de réduction un dérivé d'acide aminé cétonique correspondant à la formule I, mais contenant à la place d'un groupe $CH(NH_2)$ un groupe CO
ou en ce que l'on fait réagir un acide carboxylique de formule II

$$X - G^1 - OH \qquad II$$

où
$G^1$ représente
(a) $Z^1$
(b) Z
(c) Z-W
(d) Z-W-E

avec une amine de formule III

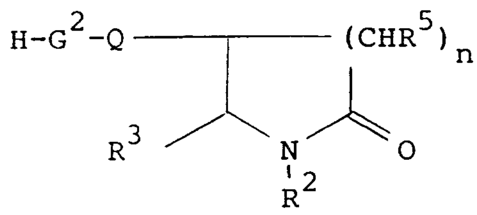

III

où

$G^2$ représente

(a) $Z^2$-W-E

(b) W-E

(c) E

(d) manque et

$Z^1$ - $Z^2$ représentent ensemble Z

et en ce que l'on libère éventuellement dans un composé de formule I un groupe amino et/ou hydroxy fonctionnellement modifié par traitement avec des agents solvolysants ou hydrogénolysants et/ou en ce que l'on transforme un composé de formule I par traitement avec un acide ou une base en l'un de ses sels.

4. Procédé pour la préparation de compositions pharmaceutiques, caractérisé en ce que l'on met un composé de formule I et/ou l'un de ses sels physiologiquement acceptables associé à au moins un support ou à un adjuvant solide, liquide ou semi-liquide et éventuellement en combinaison avec une ou plusieurs autre(s) substance(s) active(s) sous une forme de dosage appropriée.

5. Préparation pharmaceutique, caractérisée en ce qu'elle contient au moins un composé de formule I selon la revendication 1 et/ou l'un de ses sels physiologiquement acceptables.

6. Composés de formule I selon la revendication 1 et leurs sels physiologiquement acceptables pour le traitement des maladies.

7. Utilisation des composés de formule I selon la revendication 1 ou de leurs sels physiologiquement acceptables pour la préparation d'un médicament pour lutter contre l'hypertension dépendant de la rénine ou l'hyperaldostéronisme.